# EUROPEAN PATENT APPLICATION

(11) **EP 4 059 353 A1**
(43) Date of publication of application: **21.09.2022**
(21) Application number: 20888041.9
(22) Date of filing: 03.11.2020
(51) Int. Cl.: A23L 33/105, A23L 5/43, A23K 20/111, C07C 45/78, C07C 49/255, A61K 8/35, A61Q 19/00

(54) **HIGH-QUALITY CURCUMIN PRODUCT, PRODUCTION METHOD THEREFOR AND APPLICATION THEREOF**

(30) Priority: 12.11.2019 CN 201911100812
(71) Applicant: Chenguang Biotech Group Co., Ltd., Handan City, Hebei 057250 (CN)
(72) Inventor: QI, Lijun, Handan, Hebei 057250 (CN); PENG, Jingwei, Handan, Hebei 057250 (CN); GAO, Wei, Handan, Hebei 057250 (CN); LIAN, Yunhe, Handan, Hebei 057250 (CN); CHANG, Zhongqiang, Handan, Hebei 057250 (CN)
(74) Representative: Geskes, Christoph
(86) International application number: PCT/CN2020/126137
(87) International publication number: WO 2021/093636

(57) **Abstract**

Provided are a high-quality curcumin product, a production method therefor and an application thereof. The high-quality curcumin product has low solvent residue, good fluidity, and a high bulk density. The method for producing the product comprises the following steps: using a turmeric extract as a raw material, adding a certain proportion of a crystallization agent, dissolving at high temperature, then continuing heating or refluxing, cooling and crystallization, and then centrifuging or filtering to obtain crude curcumin, and further drying to obtain high-quality curcumin. The high-quality curcumin can be used as an additive for food, medicine, feed, cosmetics, etc.

## Description

### Cross-reference to related application

The present application claims priority to Chinese Patent Application No. 201911100812.2, entitled "high-quality curcumin product, production method therefor and application thereof', and filed on November 12, 2019, the entire disclosure of which is incorporated herein by reference in its entirety.

### Technical Field

The present invention relates to a high-quality curcumin product, production method therefor and application thereof, and particularly to a high-quality curcumin with low solvent residue, good fluidity and high bulk density, production method therefor and application of the product in the fields of food, feed, pharmaceutical preparations and cosmetics.

### Background Art

Turmeric (Curcuma longa L.) belongs to the genus Curcuma, and is often used as a flavoring and dye. Turmeric is the dried rhizome of Curcuma longa L. of the genus Curcuma of Zingiberaceae family. Turmeric grows from India and Australia, south to east Asia, north to southern Zhejiang, and has been used in Asia for a history of thousands of years. The active ingredients that can be extracted from turmeric are curcumin (3.5% to 5%) and turmeric oil (about 5%). After more than 30 years of research, it has been found that curcumin has various biological activities, such as anti-tumor, anti-virus, inhibiting Alzheimer's, anti-arthritis and the like.

At present, the common extraction processes of curcumin include organic solvent extraction, alkaline water extraction, enzymatic extraction, supercritical extraction and the like. The crude curcumin obtained by the above method often contains turmeric fatty oil, turmeric resin and the like, which makes curcumin less pure, not easy to dry, and the purity of curcumin also affects its application in food and medicine. Therefore, the crude curcumin needs to be further refined. Common refining methods include polyamide adsorption, macroporous resin adsorption, acetic acid precipitation, and recrystallization. In the process of solvent extraction or crystallization, due to the particularity of the molecular structure of curcumin, the organic solvent is encapsulated in the curcumin crystals, and cannot be completely removed. The residual organic solvent in the curcumin crystals circulating in the market is about 2000 to 3000 ppm, which directly affects the normal use of curcumin products as food additives. Curcumin can be used as a raw material to be directly filled into capsules for dietary supplements or pharmaceutical preparations. During filling or tableting process, the requirements for fluidity and bulk density of curcumin are high. At present, the production process of curcumin does not pay attention to controlling the crystallization process, resulting the phenomenon of low bulk density and poor fluidity of the product, which seriously affects the direct application of curcumin in pharmaceutical preparation fields and the like.

In view of the above-mentioned situation, in order to widen the application of curcumin crystals in industries such as food and overcome the current situation that direct application of curcumin is not effective, the present invention uses a turmeric extract as a raw material, and obtains a high-quality curcumin product through fine control on crystallization process.

In the prior art, there is currently a process of refining and purifying curcumin using a recrystallization method. For example, CN104072352A discloses the steps of performing ethanol reflux and recrystallization after obtaining a crude curcumin by using an extraction process with crushed ginger. In addition, CN102617316A discloses a method that turmeric is extracted with an organic solvent to remove heavy metals, and then a precipitate is obtained, the precipitate is heated with a water-containing polar solvent and cooled at room temperature to obtain curcumin crystals. There is reported (Lingtong CHEN, Separation and Purification of Curcumin by Cooling Crystallization, ZheJiang Chemical Industry, 2019, 50(5): 25-27) that the effects of cooling speed, stirring method, and whether to add seed crystals on a crystallization yield and crystal purity of curcumin were investigated, and it is disclosed that the optimal conditions for crystallization are as follows: cooling at a rate of 10°C/min, stirring magnetically, and adding crystal seeds.

However, the prior art has not investigated or optimized heating temperature, cooling temperature, stirring speed and the like in the crystallization process, and the formation of crystals is usually greatly interfered by external factors, and various parameters of crystallization conditions may affect the physicochemical properties of crystals. The prior art neither discloses which specific parameters in the crystallization process have specific effects on the solvent residue and the physicochemical properties of the crystal products, nor solves the technical problem of improving the solvent residue, crystal bulk density and fluidity in crystallization. Through the selection of solvent and the control of precise parameters such as cooling and holding time, stirring speed, reflux conditions and the like, the present invention reduces the residual amount of solvent in the crystallization product and improves the bulk density and fluidity of the crystallization product, and the process method is simple and efficient, suitable for industrial production, and achieves a purpose of providing high-quality curcumin additives for the fields of food, medicine, cosmetics and the like.

### Summary of the Invention

A purpose of the present invention is to provide a high-quality curcumin with a total curcumin content of 90% or more, total organic solvent residue of 50 ppm or less, fluidity of 2.5 s/g or less, bulk density of 0.3 g/ml or more, which is obtained by using a turmeric extract as a raw material through processing.

In some preferred embodiments, the high-quality curcumin has a total curcumin content of 95% or more, a total organic solvent residue of 30 ppm or less, a fluidity of 1.5 s/g or more, and a bulk density of 0.4 g/ml or more.

On the other hand, the present invention also provides a production method for the high-quality curcumin, the method comprises the following steps:
Step 1: dissolving: using a turmeric extract as a raw material, adding a certain proportion of a crystallization agent, and heating to dissolve;
Step 2: refluxing: subjecting the dissolved solution to refluxing by introducing an inert gas for a certain period of time under high temperature conditions;
Step 3: cooling for crystallization: cooling and stirring the turmeric extract solution that has been dissolved and refluxed for crystallization, and then holding the temperature at an end point temperature for a certain period of time;
Step 4: separation and drying: subjecting the crystallized feed liquid to filtration or centrifugation to obtain a semi-finished product of curcumin crystals, and drying the semi-finished product to obtain a high-quality curcumin.

A further improvement of the technical solution of the present invention lies in: in Step 1, the raw materials of the turmeric extract refer to turmeric extractum, curcumin, and turmeric oil resin with solvent residue of less than 10%, and further preferably commercially available turmeric extractum, curcumin, and turmeric oil resin with solvent residue of 50 to 50000 ppm, particularly preferably turmeric extractum, curcumin, and turmeric oil resin with solvent residue of 50 to 5000 ppm, and most preferably turmeric extractum, curcumin, and turmeric oil resin with solvent residue of 50 to 1000 ppm.

A further improvement of the technical solution of the present invention lies in: in Step 1, the crystallization agent refers to a substance for which the solubility of curcumin is significantly affected by temperature, preferably C2-C4 alcohols containing one or more hydroxyl groups, such as ethanol, propanol, n-propanol, isopropanol, butanol, propylene glycol, butylene glycol, etc., particularly preferably alcohols containing 1 to 3 hydroxyl groups, and most preferably alcohols containing 1 to 2 hydroxyl groups.

A further improvement of the technical solution of the present invention lies in: in Step 1, the certain proportion refers to a weight-to-volume ratio of the turmeric extract to the crystallization agent of 1:1 to 1:20, preferably 1:1 to 1:10, particularly preferably 1:1 to 1:8, and most preferably 1:3 to 1:5.

A further improvement of the technical solution of the present invention lies in: in Step 1, the dissolving is achieved by adding the crystallization agent to the raw materials, and heating the resultant to a certain temperature, and the heating temperature is any temperature at which the crystallization agent completely dissolves the raw materials without loss of active ingredients.

A further improvement of the technical solution of the present invention lies in: in Step 2, the inert gas is a gas other than oxygen, and preferably, the inert gas is carbon dioxide or nitrogen.

A further improvement of the technical solution of the present invention lies in: in Step 2, the refluxing is performed for 0.5 to 5 h, preferably 0.5 to 3 h, and particularly preferably 1 to 2 h.

A further improvement of the technical solution of the present invention lies in: in Step 3, the end point temperature is 0°C to 40°C, preferably 10°C to 30°C and particularly preferably 10°C to 20°C_{∘}

A further improvement of the technical solution of the present invention lies in: in Step 3, the stirring is performed at a speed of 5 to 100 r/min, preferably 5 to 50 r/min, and particularly preferably 5 to 30 r/min.

A further improvement of the technical solution of the present invention lies in: in Step 3, the time for holding temperature is 0 to 48 h, preferably 10 to 48 h, and particularly preferably 24 to 48 h.

The most preferred technical solution of the present invention is as follows:
Step 1: dissolving: using a turmeric extract as a raw material, adding a certain proportion of a crystallization agent, and heating to dissolve, wherein the crystallization agent is selected from ethanol, propanol, n-propanol, isopropanol, butanol, propylene glycol, and butylene glycol;
Step 2: refluxing: subjecting the dissolved solution to heating or refluxing for 1 to 2 h by introducing nitrogen or nitrogen dioxide under high temperature conditions;
Step 3: cooling for crystallization: cooling and stirring the turmeric extract solution that has been dissolved and refluxed for crystallization, and then holding the temperature at an end point temperature of 10°C to 20°C for 24 to 48 h, wherein the stirring is performed at a speed of 5 to 30 r/min;
Step 4: subjecting the feed liquid containing crystals obtained in Step 3 to filtration or centrifugation, and then drying to obtain a curcumin extract.

The high-quality curcumin of the present invention is suitable for food additives, feed additives, and suitable as a component for preparing medicines, cosmetics and food additive products.

Typical uses in the food field are for example for the colouring of functional drinks, dairy products, ice cream and the colouring of milk powders, egg products, bakery mixes and confectionery.

In a feed field, curcumin can be used, for example, in poultry breeding, or as pet functional feed additives.

In a cosmetic field, curcumin can be used, for example, as a colorant for decorative body care compositions.

In a pharmaceutical field, curcumin can be used in pharmaceutical preparations, especially tablets, coated tablets and capsules.

The beneficial effects of the present invention are as follows: the present invention effectively reduces the solvent residue in curcumin, improves the fluidity of curcumin through crystallization control, and enables curcumin to be directly used as a food additive in food. In the whole process, the effective content loss of curcumin is less than 5%, which can effectively improve the unit purity of curcumin to 90% or more. Only alcoholic solvents are used in the technological process, which effectively improves the safety of the production process and food safety. the operation of the present invention is relatively simple, and it is easy to achieve large-scale production.

### Specific Modes for Carrying Out the Embodiments

The method of the present invention is described below through specific embodiments. In addition, the embodiments are to be understood as illustrative, rather than limiting the scope of the present invention, and the spirit and scope of the present invention is limited only by the claims.

In the Examples, the solvent residues of the raw material turmeric extract and the product curcumin were determined according to the detection method of "GB1886.76-2016".

The fluidity of curcumin products was measured using a powder flow meter (Hall current meter). The fluidity of curcumin described in present application is represented by the time required for a certain amount of curcumin to flow through a standard funnel with a specified aperture, and the unit used is s/g, and the lower the numerical value, the better the fluidity of the powder.

The bulk density of curcumin products is determined according to the detection method of "GB/T 20316.2-2006".

The content of curcumin in the product is determined according to the detection method of "US Pharmacopoeia USP39-Curcumin".

### Example 1

Dissolving: 100 g of turmeric extractum raw material with a solvent residue of 5% (mass fraction) was taken, and 100 ml of n-butanol was added, and the resultant was heated up to 115°C, and stirred to dissolve;
Refluxing: carbon dioxide was introduced at 117°C, and cooling was performed after refluxingfor 0.5 h;
Cooling for crystallization: the refluxed solution was cooled and stirred at a speed of 100 r/min for crystallization, wherein the temperature was cooled to 40°C, and then held for 48 h;
Separating and drying: the crystallized feed liquid was suction filtered to obtain a semi-finished product of curcumin crystals, and the semi-finished product was dried to obtain a high-quality curcumin.
Through the above production method for a high-quality curcumin, the obtained curcumin has a solvent residue of 48 ppm, a fluidity of 2.5 s/g, a bulk density of 0.32 g/ml, and a curcumin content of 90.3%.

### Example 2

Dissolving: 200 g of turmeric extractum raw material with a solvent residue of 1% (mass fraction) was taken, and 400 ml of propylene glycol was added, and the resultant was heated up to 150°C, and stirred to dissolve;
Refluxing: carbon dioxide was introduced at 150°C, and cooling was performed after heating for 1 h;
Cooling for crystallization: the refluxed solution was cooled and stirred at a speed of 50 r/min for crystallization, wherein the temperature was cooled to 30°C, and then held for 24 h;
Separating and drying: the crystallized feed liquid was suction filtered to obtain a semi-finished product of curcumin crystals, and the semi-finished product was dried to obtain a high-quality curcumin.

Through the above production method for a high-quality curcumin, the obtained curcumin has a solvent residue of 42 ppm, a fluidity of 2.0 s/g, a bulk density of 0.37 g/ml, and a curcumin content of 93.7%.

### Example 3

Dissolving: 150 g of turmeric extract raw material with a solvent residue of 5000 ppm was taken, and 1500 ml of ethanol was added, and the resultant was heated up to 78°C, and stirred to dissolve;
Refluxing: nitrogen was introduced at 75°C, and cooling was performed after refluxing for 2 h;
Cooling for crystallization: the refluxed solution was cooled and stirred at a speed of 30 r/min for crystallization, wherein the temperature was cooled to 20°C, and then held for 12 h;
Separating and drying: the crystallized feed liquid was suction filtered to obtain a semi-finished product of curcumin crystals, and the semi-finished product was dried to obtain a high-quality curcumin.

Through the above production method for a high-quality curcumin, the obtained curcumin has a solvent residue iof 30 ppm, a fluidity of 1.5 s/g, a bulk density of 0.45 g/ml, and a curcumin content of 95.6%.

### Example 4

Dissolving: 100 g of turmeric extract raw material with a solvent residue of 1000 ppm was taken, and 2000 ml of isopropanol was added, and the resultant was heated up to 82°C, and stirred to dissolve;
Refluxing: carbon dioxide was introduced at 82°C, and cooling was performed after refluxing for 5 h;
Cooling for crystallization: the refluxed solution was cooled and stirred at a speed of 10 r/min for crystallization, wherein the temperature was cooled to 10°C, and then held for 1 h;
Separating and drying: the crystallized feed liquid was suction filtered to obtain a semi-finished product of curcumin crystals, and the semi-finished product was dried to obtain a high-quality curcumin.

Through the above production method for a high-quality curcumin, the obtained curcumin has a solvent residue of 25 ppm, a fluidity of 1.0 s/g, a bulk density of 0.50 g/ml, and a curcumin content of 97.0%.

### Example 5

Dissolving: 100 g of turmeric extract raw material with a solvent residue of 50 ppm was taken, and 500 ml of propylene glycol was added, and the resultant was heated up to 100°C, and stirred to dissolve;
Refluxing: nitrogen was introduced at 100°C, and cooling was performed after heating for 5 h;
Cooling for crystallization: the refluxed solution was cooled and stirred at a speed of 5 r/min for crystallization, wherein the temperature was cooled to 15°C, and then held for 12 h;
Separating and drying: the crystallized feed liquid was suction filtered to obtain a semi-finished product of curcumin crystals, and the semi-finished product was dried to obtain a high-quality curcumin.

Through the above production method for a high-quality curcumin, the obtained curcumin has a solvent residue of 15 ppm, a fluidity of 0.5 s/g, a bulk density of 0.55 g/ml, and a curcumin content of 98.7%.

### Comparative Experiment:

Except for the experimental parameter values clearly listed in Table 1, other experimental parameters of Comparative Examples 1-7 are the same as those of Example 3. Table 1 shows performance indicators of the turmeric extract obtained under different experimental conditions.

**Table 1**

| Sample No. | Experimental conditions | Performance indicators of the obtained extract | | | |
|---|---|---|---|---|---|
| | | Solvent residual content/ppm | Fluidity/(s/g) | Bulk density/(g/ml) | Curcumin content/% |
| Experimental Example 1 | Exactly the same as conditional parameters of Example 3 | 30 | 1.5 | 0.45 | 95.6 |
| Comparative Example 1 | Temperature for dissolving is 58°C | 215 | 3.3 | 0.29 | 88 |
| Comparative Example 2 | Temperature for dissolving is 120°C | 75 | 2.8 | 0.38 | 85 |
| Comparative Example 3 | No nitrogen was introduced during the dissolving and heating process | 133 | 4.0 | 0.33 | 90 |
| Comparative Example 4 | Crystallization process without stirring | 147 | 3.5 | 0.37 | 93.8 |
| Comparative Example 5 | Crystallization process was performed under stirring at 200 r/min | 176 | 3.8 | 0.24 | 92.7 |
| Comparative Example 6 | End point temperature of cooling is 50°C | 243 | 3.2 | 0.30 | 93.2 |
| Comparative Example 7 | End point temperature of cooling is 0°C | 50 | 3.5 | 0.27 | 91.3 |

It can be seen from the above Comparative Examples that the selection of various crystallization condition parameters claimed in the present application significantly improves the solvent residual content in the crystals, fluidity and bulk density of the crystals.

Although the present invention has been described in detail above with general description, specific embodiments and experiments, it is obvious to a person skilled in the art that some modifications or improvements can be made on the basis of the present invention. Therefore, these modifications or improvements made without departing from the spirit of the present invention fall within the scope of the claimed protection of the present invention.

### Industrial applicability

The present invention provides a high-quality curcumin product, a production method therefor and an application thereof. The high-quality curcumin product has low solvent residue, good fluidity, and a high bulk density. The method for producing the product comprises the following steps of: using a turmeric extract as a raw material, adding a certain proportion of a crystallization agent, dissolving at high temperature, then continuing heating or refluxing, cooling for crystallization, and then centrifuging or filtering to obtain crude curcumin, and further drying to obtain a high-quality curcumin. The high-quality curcumin can be used as an additive for food, medicine, feed, cosmetics and the like, and has good economic value and application prospects.

## Claims

1. A curcumin extract, wherein a total curcumin content is 90% or more, and a total solvent residue is 50 ppm or less.

2. The curcumin extract according to claim 1, wherein a fluidity is 2.5 s/g or less.

3. The curcumin extract according to claim 1 or 2, wherein a bulk density is 0.3 g/ml or more.

4. A production method for the curcumin extract according to any one of claims 1 to 3, **characterized by** comprising the following processing steps:
Step 1: dissolving: using a turmeric extract as a raw material, adding a certain proportion of a crystallization agent, and heating to dissolve;
Step 2: refluxing: subjecting the dissolved solution to heating or refluxing by introducing an inert gas for a certain period of time under high temperature conditions;
Step 3: cooling for crystallization: cooling and stirring the turmeric extract solution that has been dissolved and refluxed for crystallization, and then holding the temperature at an end point temperature for a certain period of time, a crystal obtained in Step 3 is a curcumin extract.

5. The production method according to claim 4, wherein the curcumin extract in step 3 is obtained by subjecting feed liquid containing crystals to filtration or centrifugation and then drying.

6. The production method according to claim 4 or 5, wherein, in step 1, the raw material of a turmeric extract refers to turmeric extractum, curcumin, and turmeric oil resin with solvent residue of less than 10% (mass fraction), and further preferably commercially available turmeric extractum, curcumin, and turmeric oil resin with solvent residue of 50 to 50000 ppm, particularly preferably turmeric extractum, curcumin, and turmeric oil resin with solvent residue of 50 to 5000 ppm, and most preferably turmeric extractum, curcumin, and turmeric oil resin with solvent residue of 50 to 1000 ppm.

7. The production method according to any one of claims 4 to 6, wherein the crystallization agent in Step 1 can dissolve curcumin and crystallize curcumin under conditions of temperature change, the crystallization agent is preferably C2-C4 alcohols containing one or more hydroxyl groups, particularly preferably alcohols containing 1 to 3 hydroxyl groups, and most preferably alcohols containing 1 to 2 hydroxyl groups.

8. The production method according to any one of claims 4 to 7, wherein the certain proportion in Step 1 refers to a weight-to-volume ratio of the turmeric extract to the crystallization agent of 1:1 to 1:20, preferably 1:1 to 1:10, particularly preferably 1:1 to 1:8, and most preferably 1:3 to 1:5.

9. The production method according to any one of claims 4 to 8, wherein the inert gas in Step 2 is a gas other than oxygen, and preferably, the inert gas is carbon dioxide or nitrogen.

10. The production method according to any one of claims 4 to 9, wherein the heating or refluxing in Step 2 is performed for 0.5 to 5 h, preferably the refluxing is performed for 0.5 to 3 h, and particularly preferably the refluxing is performed for 1 to 2 h.

11. The production method according to any one of claims 4 to 10, wherein the end point temperature in Step 3 is 0°C to 40°C, preferably 10°C to 30°C and particularly preferably 10°C to 20°C.

12. The production method according to any one of claims 4 to 11, wherein the stirring in Step 3 is performed at a speed of 5 to 100 r/min, preferably 5 to 50 r/min, and particularly preferably 5 to 30 r/min.

13. The production method according to any one of claims 4 to 12, wherein the time for holding temperature in Step 3 is 0 to 48 h, preferably 10 to 48 h, and particularly preferably 24 to 48 h.

14. A curcumin extract prepared by the production method according to any one of claims 4 to 13, wherein, in the extract, a total curcumin content is 90% or more, a total solvent residue is 50 ppm or less, the fluidity of the extract is 2.5 s/g or less, and the bulk density of the extract is 0.3 g/ml or more.

15. A composition, comprising the curcumin extract of claim 1, 2, 3 or 14.

16. Use of the curcumin extract of claim 1, 2, 3 or 14 as a functional additive or colorant for food, feed, pharmaceutical preparations and cosmetics.
